# FASCICULE DE BREVET EUROPEEN

(11) **EP 2 675 336 B1**
(45) Date de publication et mention de la délivrance du brevet: **19.12.2018**
(21) Numéro de dépôt: 12702269.7
(22) Date de dépôt: 03.02.2012
(51) Int. Cl.: A61B 17/42, A61B 1/303, A61B 1/32, A61B 17/02, A61M 29/00

(54) **SPECULUM VAGINAL A QUATRE VALVES**
VAGINALSPEKULUM MIT VIER VENTILEN
FOUR-VALVE VAGINAL SPECULUM

(30) Priorité: 14.02.2011 FR 1151188
(43) Date de publication de la demande: 25.12.2013
(73) Titulaire: Assistance Publique - Hôpitaux de Paris, 75004 Paris (FR)
(72) Inventeur: COHEN, Jonathan, 75020 Paris (FR)
(74) Mandataire: Lebkiri, Alexandre
(86) Numéro de dépôt international: PCT/EP2012/051879
(87) Numéro de publication internationale: WO 2012/110334

(56) Documents cités:
- EP-A2- 1 195 141
- WO-A1-2009/158435
- US-A- 1 963 173
- US-A- 4 702 230
- US-A- 4 726 356
- US-A- 4 989 587
- US-A1- 2005 228 232
- US-A1- 2006 025 656
- US-A1- 2010 113 885
- US-A1- 2010 217 091

## Description

### DOMAINE TECHNIQUE DE L'INVENTION

La présente invention concerne un speculum vaginal à quatre valves.

Le domaine technique est, d'une façon générale, celui des dispositifs à valves vaginales destinés à écarter les parois du vagin. Il est particulièrement adapté à une exploration vaginale ou une chirurgie vaginale en post-partum immédiat, c'est-à-dire dans les vingt-quatre heures suivant un accouchement. Cependant il peut également être utilisé en dehors du post-partum dans toute sorte de chirurgie vaginale, par exemple une hystérectomie par voie vaginale, un cerclage, une conisation, une vaporisation laser du col, une chirurgie du prolapsus, ou encore une suture de plaies vaginales

Le spéculum selon l'invention est utilisé notamment, mais non exclusivement, en cas d'hémorragie vaginale pour rechercher une lésion cervicale ou vaginale responsable des saignements. Il s'agit d'un examen fondamental qui ne doit pas être négligé, afin de ne pas traiter inutilement ou sans efficacité une cause utérine de l'hémorragie.

### ETAT DE LA TECHNIQUE ANTERIEUR

Actuellement, un obstétricien effectuant une exploration vaginale ou une chirurgie vaginale en post-partum immédiat, est aidé d'un assistant qui maintient deux valves vaginales de part et d'autre des parois du vagin. Or en cas d'urgence, un assistant n'est pas toujours disponible.

Par ailleurs, des speculums vaginaux de type Cusco™ ou Collin™ ne sont pas adaptés au post-partum immédiat. Ces spéculums comportent deux branches articulées l'une sur l'autre par une extrémité, chaque branche étant rectiligne et ayant une section arquée de manière à présenter une surface externe convexe et une surface interne concave, des moyens étant prévus pour maintenir un écart angulaire entre les deux branches. Ces branches sont généralement appelées valves. L'obstétricien les insère dans le vagin lors d'un examen avant d'augmenter leur écart angulaire. Or en post-partum immédiat, le vagin a subi une importante distension. Les dimensions de ces spéculums ne sont alors plus adaptées pour maintenir suffisamment écartées les parois du vagin distendu, ni pour se maintenir seul en position dans le vagin.

### DESCRIPTION GENERALE DE L'INVENTION

L'objet de l'invention propose un instrument à valves vaginales apte à écarter des parois vaginales, notamment en post-partum immédiat, et à les maintenir en position sans intervention extérieure.

L'invention concerne donc essentiellement un speculum vaginal comprenant :
- une première tige supportant :
   - une première branche apte à coulisser le long de la première tige, une première valve étant fixée à la première branche ;
   - une deuxième branche, une deuxième valve étant fixée à la deuxième branche ;
- une deuxième tige supportant :
   - une troisième branche apte à coulisser le long de la deuxième tige, une troisième valve étant fixée à la troisième branche ;
   - une quatrième branche, une quatrième valve étant fixée à la quatrième branche ;
la première tige et la deuxième tige étant solidaires l'une de l'autre.

Un obstétricien utilisant le spéculum vaginal selon l'invention pour effectuer une exploration vaginale ou une chirurgie vaginale, place les quatre valves à l'intérieur du vagin, et les écarte les unes des autres grâce à l'aptitude de la première branche et de la troisième branche à coulisser respectivement le long de la première tige et la deuxième tige, écartant ainsi les parois du vagin.

Grâce à l'invention, les parois vaginales sont maintenues écartées, et le spéculum se maintient seul en position dans le vagin. Un assistant n'est donc pas nécessaire, et l'instrument est utilisable en post-partum immédiat.

Outre les caractéristiques qui viennent d'être évoquées dans le paragraphe précédent, le spéculum vaginal selon l'invention peut présenter une ou plusieurs caractéristiques complémentaires parmi les suivantes, considérées individuellement ou selon toutes les combinaisons techniquement possibles :
- la première valve et la deuxième valve sont planes et s'étendent selon des plans inclinés l'un par rapport à l'autre d'un angle compris entre 0 et 30 degrés, et de préférence compris entre 5 et 20 degrés. Un tel agencement améliore le maintien du spéculum selon l'invention sur le vagin.
- la troisième valve et la quatrième valve s'étendent selon des plans inclinés l'un par rapport à l'autre d'un angle compris entre 0 et 30 degrés, et de préférence compris entre 5 et 20 degrés. Un tel agencement améliore le maintien du spéculum selon l'invention sur le vagin.
- la première tige est pourvue d'un premier système de crémaillère apte à faire coulisser la première branche le long de la première tige. Un tel agencement est particulièrement pratique d'utilisation pour un obstétricien utilisant le spéculum selon l'invention. En effet la première branche est apte à coulisser librement le long de la première tige dans un sens dans lequel la première valve et la deuxième valve s'écartent l'une de l'autre, alors qu'un coulissement en sens inverse est bloqué, avantageusement par un clapet anti-retour.
- la deuxième tige est pourvue d'un deuxième système de crémaillère apte à faire coulisser la troisième branche le long de la deuxième tige. Un tel agencement est particulièrement pratique d'utilisation pour un obstétricien utilisant le spéculum selon l'invention. En effet la troisième branche est apte à coulisser librement le long de la deuxième tige dans un sens dans lequel la troisième valve et la quatrième valve s'écartent l'une de l'autre, alors qu'un coulissement en sens inverse est bloqué, avantageusement par un clapet anti-retour.
- le spéculum vaginal comporte un premier moyen de fixation de la position de la première branche sur la première tige. Ainsi un obstétricien utilisant le spéculum selon l'invention pour maintenir écartées des parois d'un vagin, est apte, après avoir inséré les quatre valves dans le vagin, et déterminé un écartement optimal entre la première valve et de la deuxième valve, à figer cet écartement en fixant la position de la première branche sur la première tige.
- le spéculum vaginal comporte un deuxième moyen de fixation de la position de la troisième branche sur la deuxième tige. Ainsi un obstétricien utilisant le spéculum selon l'invention pour maintenir écartées des parois d'un vagin, est apte, après avoir inséré les quatre valves dans le vagin, et déterminé un écartement optimal de la troisième valve et de la quatrième valve, à figer cet écartement en fixant la position de la première branche sur la première tige.
- la première tige et la deuxième tige s'étendent selon des directions sécantes, et de préférence sensiblement orthogonales. Une telle disposition est particulièrement avantageuse pour améliorer le maintien du spéculum selon l'invention sur le vagin.
- la première branche et la première tige s'étendent selon des directions sensiblement orthogonales. Une telle disposition est particulièrement avantageuse pour améliorer le maintien du spéculum selon l'invention sur le vagin.
- la deuxième branche et la première tige s'étendent selon des directions sensiblement orthogonales. Une telle disposition est particulièrement avantageuse pour améliorer le maintien du spéculum selon l'invention sur le vagin.
- la troisième branche et la deuxième tige s'étendent selon des directions sensiblement orthogonales. Une telle disposition est particulièrement avantageuse pour améliorer le maintien du spéculum selon l'invention sur le vagin.
- la quatrième branche et la deuxième tige s'étendent selon des directions sensiblement orthogonales. Une telle disposition est particulièrement avantageuse pour améliorer le maintien du spéculum selon l'invention sur le vagin.
- selon un mode de réalisation, le spéculum vaginal selon l'invention est en métal. Dans ce mode de réalisation, le spéculum est robuste et réutilisable.
- selon un autre mode de réalisation, le spéculum vaginal selon l'invention est en plastique. Dans ce mode de réalisation, le spéculum est à usage unique, et avantageusement pré-stérilisé. Il n'a pas besoin d'être stérilisé avant chaque examen.
- selon un mode de réalisation, les valves sont pleines. Des valves pleines permettent de comprimer les parois vaginales afin de réaliser une éventuelle hémostase mécanique d'un saignement vaginal causé par une déchirure spontanée ou par une épisiotomie. - selon un autre mode de réalisation, les valves sont creuses. Des valves creuses offrent une plus grande visibilité sur les parois intérieures du vagin. Elles sont plus adaptées pour réaliser des sutures de la muqueuse vaginale en cas de nécessité.

L'invention et ses différentes applications seront mieux comprises à la lecture de la description qui suit et à l'examen des figures qui l'accompagnent.

### BREVE DESCRIPTION DES FIGURES

Les figures ne sont présentées qu'à titre indicatif et nullement limitatif de l'invention. Les figures montrent :
- à la figure 1, une représentation en perspective d'un spéculum vaginal selon un mode de réalisation de l'invention ;
- à la figure 2, une représentation en vue de face dudit spéculum ;
- à la figure 3, une représentation en vue de dessus dudit spéculum.

### DESCRIPTION DETAILLEE D'AU MOINS UN MODE DE REALISATION DE L'INVENTION

Sauf précision contraire, un même élément apparaissant sur des figures différentes présente une référence unique.

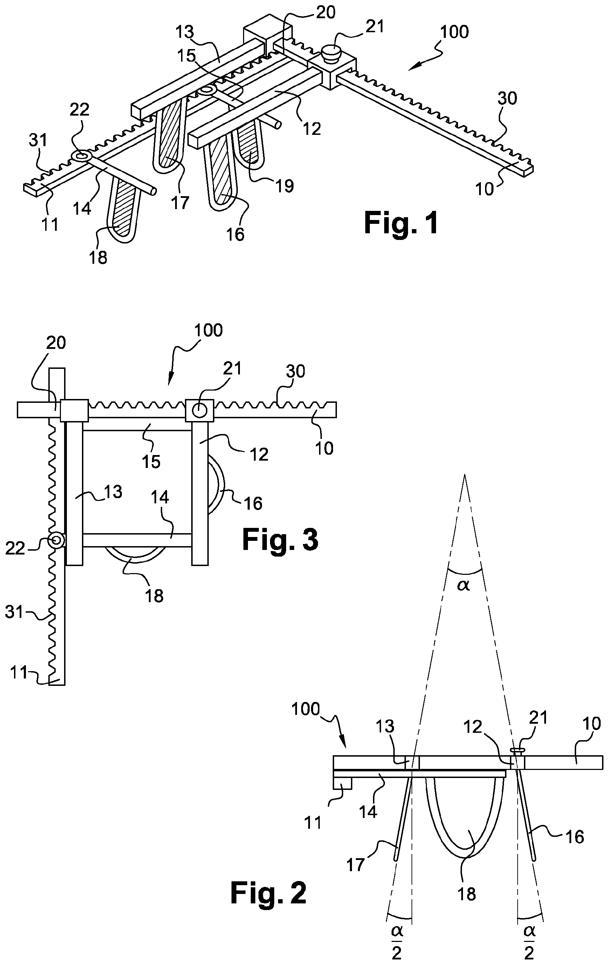

La figure 1 est une représentation en perspective d'un spéculum vaginal 100 à quatre valves selon un mode de réalisation non limitatif de l'invention.
Le spéculum vaginal 100 comporte :
- une première tige 10
- une deuxième tige 11
- un moyen de solidarisation 20 de la première tige 10 et de la deuxième tige 11
- une première branche 12
- une deuxième branche 13
- une troisième branche 14
- une quatrième branche 15
- une première valve 16
- une deuxième valve 17
- une troisième valve 18
- une quatrième valve 19
- un premier moyen de fixation 21 de la position de la première branche 12 sur la première tige 10
- un deuxième moyen de fixation 22 de la position de la troisième branche 14 sur la deuxième tige 11
- un premier système de crémaillère 30
- un deuxième système de crémaillère 31

La première tige 10 et la deuxième tige 11 sont solidaires l'une de l'autre par le moyen de solidarisation 20. Le moyen de solidarisation 20 est par exemple une soudure si le spéculum vaginal 100 est constitué de métal. La solidarisation peut également être obtenue par vissage, ou bien par collage, notamment si le spéculum vaginal 100 est en plastique. Dans un autre mode de réalisation de l'invention, la première tige 10 et la deuxième tige 11 sont faites d'un seul tenant.

La première branche 12 et la deuxième branche 13 sont supportées par la première tige 10. Dans le mode de réalisation non limitatif décrit, la première branche 12 et la deuxième branche 13 s'étendent selon des directions parallèles, et ladite direction est orthogonale à la direction selon laquelle s'étend la première tige 10. La troisième branche 14 et la quatrième branche 15 sont supportées par la deuxième tige 11. Dans le mode de réalisation non limitatif décrit, la troisième branche 14 et la quatrième branche 15 s'étendent selon des directions parallèles, et ladite direction est orthogonale à la direction selon laquelle s'étend la deuxième tige 11.

La première valve 16, la deuxième valve 17, la troisième valve 18 et la quatrième valve 19 sont fixées respectivement à la première branche 12, la deuxième branche 13, la troisième branche 14 et la quatrième branche 15. On définit deux types de valves : des valves pleines et des valves creuses. Une valve est constituée d'un pourtour fermé et d'un espace intérieur, l'espace intérieur étant délimité par le pourtour. Quand l'espace intérieur est vide, on parle de valve vide, et quand l'espace intérieur est intégralement constitué de matière, on parle de valve pleine. Dans le mode de réalisation non limitatif décrit, les quatre valves sont pleines, mais selon d'autres modes de réalisations, au moins une valve est creuse.

La première branche 12 est apte à coulisser le long de la première tige 10. Le premier moyen de fixation 21 de la position de la première branche 12 sur la première tige 10 est apte à bloquer le coulissement de la première branche 12 le long de la première tige 10. De même, la troisième branche 14 est apte à coulisser le long de la deuxième tige 11. Le deuxième moyen de fixation 22 de la position de la troisième branche 14 sur la deuxième tige 11 est apte à bloquer le coulissement de la troisième branche 14 le long de la deuxième tige 11.

Dans un autre mode de réalisation de l'invention, la deuxième branche 13 est également apte à coulisser le long de la première tige 10, et la quatrième branche 15 est également apte à coulisser le long de la deuxième tige 11.

La figure 2 est une représentation en vue de dessus du spéculum vaginal 100. Dans le mode de réalisation décrit, les valves sont planes et s'étendent des plans inclinés l'un par rapport à l'autre d'un angle a. Selon un mode de réalisation non limitatif de l'invention, l'écart angulaire entre le plan selon lequel s'étend la première valve 16, et un plan orthogonal à la direction selon laquelle s'étend la première tige 10, vaut α/2. L'écart angulaire entre le plan selon lequel s'étend la deuxième valve 17, et un plan orthogonal à la direction selon laquelle s'étend la première tige 10, vaut alors également α/2. a est avantageusement compris entre 0 et 30 degrés, et de préférence compris entre 5 et 20 degrés. Dans le mode de réalisation non limitatif représenté à la figure 2, α vaut 20°.

La figure 3 est une représentation en vue de dessus du spéculum vaginal 100. La première tige 10 est munie d'un premier système de crémaillère 30, et la deuxième tige 11 est munie d'un deuxième système de crémaillère 31. Un tel système est particulièrement pratique d'utilisation pour un obstétricien utilisant le spéculum vaginal 100 selon l'invention. En effet, grâce à ce système, la première branche 21 est apte à coulisser librement le long de la première tige 10 dans un sens dans lequel la première valve 16 et la deuxième valve 17 s'écartent l'une de l'autre, et la troisième branche 14 est apte à coulisser librement le long de la deuxième tige 11 dans un sens dans lequel la troisième valve 18 et la quatrième valve 19 s'écartent l'une de l'autre, alors que les coulissements en sens inverses sont bloqués. Ces coulissements en sens inverses sont bloqués par exemple par deux clapets anti-retour, et débloqués lorsque l'obstétricien actionne les clapets anti-retour.

Dans un tel mode de réalisation, les deux clapets anti-retour constituent le premier moyen de fixation 21 de la position de la première branche 12 sur la première tige 10 et le deuxième moyen de fixation 22 de la position de la troisième branche 14 sur la deuxième tige 11.

Un obstétricien utilisant le spéculum vaginal 100 pour effectuer une exploration vaginale ou une chirurgie vaginale en post-partum immédiat, place les quatre valves à l'intérieur du vagin, et les écarte les unes des autres grâce à l'aptitude de la première branche et de la troisième branche à coulisser le long de respectivement la première tige et la deuxième tige, écartant ainsi les parois du vagin. Lorsque l'obstétricien a déterminé un écartement des valves qui lui convient, il fixe la position des valves par le premier moyen de fixation 21 de la position de la première branche 12 sur la première tige 10 et le deuxième moyen de fixation 22 de la position de la troisième branche 14 sur la deuxième tige 11. Le spéculum vaginal 100 se maintient alors en position dans le vagin sans intervention extérieure, et l'obstétricien dispose donc de ses deux mains pour effectuer une exploration vaginale ou une opération vaginale.

## Revendications

1. Speculum vaginal comprenant :
- une première tige (10) pourvue d'un premier système de crémaillère (30), supportant :
▪ une première branche (12) apte à coulisser le long de la première tige (10) via le premier système de crémaillère (30), une première valve (16) étant fixée à la première branche (12), un premier moyen de fixation (21) étant apte à bloquer le coulissement de la première branche (12) le long de la première tige (10) ;
▪ une deuxième branche (13), une deuxième valve (17) étant fixée à la deuxième branche (13) ;
- une deuxième tige (11) pourvue d'un deuxième système de crémaillère (31), supportant :
▪ une troisième branche (14) apte à coulisser le long de la deuxième tige (11) via le deuxième système de crémaillère (31), une troisième valve (18) étant fixée à la troisième branche (14), un deuxième moyen de fixation (22) étant apte à bloquer le coulissement de la troisième branche (14) le long de la deuxième tige (11) ;
▪ une quatrième branche (15), une quatrième valve (19) étant fixée à la quatrième branche (15) ;
la première tige (10) et la deuxième tige (11) étant solidaires l'une de l'autre.

2. Spéculum vaginal selon la revendication précédente, la première valve (16) et la deuxième valve (17) étant planes et s'étendant selon des plans inclinés l'un par rapport à l'autre d'un angle α compris entre 0 et 30 degrés, et de préférence compris entre 5 et 20 degrés.

3. Spéculum vaginal selon l'une des revendications précédentes, la troisième valve (18) et la quatrième valve (19) s'étendant selon des plans inclinés l'un par rapport à l'autre d'un angle α compris entre 0 et 30 degrés, et de préférence compris entre 5 et 20 degrés.

4. Spéculum vaginal selon l'une des revendications précédentes, la première tige (10) et la deuxième tige (11) s'étendant selon des directions sécantes, et de préférence sensiblement orthogonales.

5. Spéculum vaginal selon l'une des revendications précédentes:
- la première branche (12) et la première tige (10) s'étendant selon des directions sensiblement orthogonales ;
- la deuxième branche (13) et la première tige (10) s'étendant selon des directions sensiblement orthogonales ;
- la troisième branche (14) et la deuxième tige (11) s'étendant selon des directions sensiblement orthogonales ;
- la quatrième branche (15) et la deuxième tige (11) s'étendant selon des directions sensiblement orthogonales.

6. Spéculum vaginal selon l'une des revendications précédentes, étant en métal.

7. Spéculum vaginal selon l'une des revendications 1 à 5, étant en plastique.

8. Spéculum vaginal selon l'une des revendications précédentes, les valves étant pleines.

9. Spéculum vaginal selon l'une des revendications 1 à 7, les valves étant creuses.

## Patentansprüche

1. Vaginalspekulum, umfassend:
- einen ersten Stab (10), der mit einem ersten Zahnstangensystem (30) versehen ist, das trägt:
▪ einen ersten Zweig (12), der geeignet ist, entlang des ersten Stabes (10) über das erste Zahnstangensystem (30) zu gleiten, wobei ein erstes Ventil (16) an dem ersten Zweig (12) befestigt ist, wobei ein erstes Befestigungsmittel (21) geeignet ist, das Gleiten des ersten Zweigs (12) entlang des ersten Stabes (10) zu blockieren;
▪ einen zweiten Zweig (13), wobei ein zweites Ventil (17) an dem zweiten Zweig (13) befestigt ist;
- einen zweiten Stab (11), der mit einem zweiten Zahnstangensystem (31) versehen ist, das trägt:
▪ einen dritten Zweig (14), der geeignet ist, entlang des zweiten Stabes (11) über das zweite Zahnstangensystem (31) zu gleiten, wobei ein drittes Ventil (18) an dem dritten Zweig (14) befestigt ist, wobei ein zweites Befestigungsmittel (22) geeignet ist, das Gleiten des dritten Zweigs (14) entlang des zweiten Stabes (11) zu blockieren;
▪ einen vierten Zweig (15), wobei ein viertes Ventil (19) an dem vierten Zweig (15) befestigt ist;
wobei der erste Stab (10) und der zweite Stab (11) fest miteinander befestigt sind.

2. Vaginalspekulum gemäß dem voranstehenden Anspruch, wobei das erste Ventil (16) und das zweite Ventil (17) eben sind und sich gemäß im Verhältnis zueinander um einen zwischen 0 und 30 Grad inbegriffenen und bevorzugt zwischen 5 und 20 Grad inbegriffenen Winkel α geneigten Ebene erstrecken.

3. Vaginalspekulum gemäß einem der voranstehenden Ansprüche, wobei sich das dritte Ventil (18) und das vierte Ventil (19) gemäß im Verhältnis zueinander um einen zwischen 0 und 30 Grad inbegriffenen und bevorzugt zwischen 5 und 20 Grad inbegriffenen Winkel α geneigten Ebene erstrecken.

4. Vaginalspekulum gemäß einem der voranstehenden Ansprüche, wobei sich der erste Stab (10) und der zweite Stab (11) gemäß sich schneidenden und bevorzugt deutlich orthogonalen Richtungen erstrecken.

5. Vaginalspekulum gemäß einem der voranstehenden Ansprüche,
- wobei sich der erste Zweig (12) und der erste Stab (10) gemäß deutlich orthogonalen Richtungen erstrecken;
- wobei sich der zweite Zweig (13) und der erste Stab (10) gemäß deutlich orthogonalen Richtungen erstrecken;
- wobei sich der dritte Zweig (14) und der zweite Stab (11) gemäß deutlich orthogonalen Richtungen erstrecken;
- wobei sich der vierte Zweig (15) und der zweite Stab (11) gemäß deutlich orthogonalen Richtungen erstrecken.

6. Vaginalspekulum gemäß einem der voranstehenden Ansprüche aus Metall.

7. Vaginalspekulum gemäß einem der Ansprüche 1 bis 5 aus Plastik.

8. Vaginalspekulum gemäß einem der voranstehenden Ansprüche, wobei die Ventile massiv sind.

9. Vaginalspekulum gemäß einem der Ansprüche 1 bis 7, wobei die Ventile hohl sind.

## Claims

1. A vaginal speculum (100) including:
- a first rod (10), provided with a first rack system (30),supporting:
- a first arm (12) slidable along the first rod (10) via the first rack system (30), a first valve (16) being attached to the first arm (12), a first means (21) being able to lock the sliding of the first arm (12) along the first rod (10);
- a second arm (13), a second valve (17) being attached to the second arm (13);
- a second rod (11), provided with a second rack system (31), supporting:
- a third arm (14) slidable along the second rod (11) via the second rack system (31), a third valve (18) being attached to the third arm (14), a second means (22) being able to lock the sliding of the third arm (14) along the second rod (11);
- a fourth arm (15), a fourth valve (19) being attached to the fourth arm (15);
the first rod (10) and the second rod (11) being integral with each other.

2. The vaginal speculum (100) according to the preceding claim, the first valve (16) and the second valve (17) being planar and extending along planes tilted to each other at an angle α between 0 and 30 degrees, and preferably between 5 and 20 degrees.

3. The vaginal speculum (100) according to one of the preceding claims, the third valve (18) and the fourth valve (19) extending along planes tilted to each other at an angle α between 0 and 30 degrees, and preferably between 5 and 20 degrees.

4. The vaginal speculum (100) according to one of the preceding claims, the first rod (10) and the second rod (11) extending along intersecting, and preferably substantially orthogonal, directions.

5. The vaginal speculum (100) according to one of the preceding claims,:
- the first arm (12) and the first rod (10) extending along substantially orthogonal directions;
- the second arm (13) and the first rod (10) extending along substantially orthogonal directions;
- the third arm (14) and the second rod (11) extending along substantially orthogonal directions;
- the fourth arm (15) and the second rod (11) extending along substantially orthogonal directions.

6. The vaginal speculum (100) according to one of the preceding claims, being of metal.

7. The vaginal speculum (100) according to one of claims 1 to 5, being of plastics.

8. The vaginal speculum (100) according to one of the preceding claims, the valves being solid.

9. The vaginal speculum (100) according to one of claims 1 to 7, the valves being hollow.
